# FASCICULE DE BREVET EUROPEEN

(11) **EP 1 068 256 B1**
(45) Date de publication et mention de la délivrance du brevet: **09.04.2003**
(21) Numéro de dépôt: 98941499.0
(22) Date de dépôt: 30.07.1998
(51) Int. Cl.: C08G 77/38

(54) **PROCEDE DE PREPARATION DE SILICONES A FONCTION(S) ARYLALKYLE(S) PAR HYDROSILYLATION**
VERFAHREN ZUR HERSTELLUNG VON ARYLALKYLFUNKTIONELLEN SILICONEN DURCH HYDROSILYLIERUNG
METHOD FOR PREPARING SILICONES WITH ARYLALKYL FUNCTION(S) BY HYDROSILYLATION

(30) Priorité: 09.04.1998 FR 9804483
(43) Date de publication de la demande: 17.01.2001
(73) Titulaire: RHODIA CHIMIE, 92408 Courbevoie Cédex (FR)
(72) Inventeur: BRANLARD, Paul, F-69005 Lyon (FR); MIGNANI, Gérard, F-69008 Lyon (FR); OLIER, Philippe, F-69007 Lyon (FR); WILLEMIN, Claudie, F-75008 Paris (FR)
(74) Mandataire: Wattremez, Catherine
(86) Numéro de dépôt international: FR9801698
(87) Numéro de publication internationale: WO99052965

(56) Documents cités:
- FR-A- 2 740 037
- US-A- 5 248 789
- DATABASE WPI Week 9213 Derwent Publications Ltd., London, GB; AN 92-102367 XP002076856 & JP 04 046933 A (SHINETSU) , 17 février 1992

## Description

La présente invention a pour objet un procédé de préparation de silicones à fonction(s) arylalkyle(s) de haute pureté, par réaction d'hydrosilylation d'un hydrogénopolyorganosiloxane et d'un composé monovinylaromatique. Les silicones à fonction(s) arylalkyle(s) de haute pureté ainsi obtenus peuvent être utilisés comme agents émollients et/ou véhicules dans les compositions cosmétiques, ainsi que comme agents solubilisants et émollients dans les compositions parfumantes.

Il est connu de préparer des silicones portant des fonctions arylalkyles par réaction d'hydrosilylation d'un silicone portant au moins une fonction SiH avec un composé aromatique présentant une insaturation vinylique, en présence d'un catalyseur à base de platine notamment.
L'hydrosilylation de composés aromatiques à insaturation vinylique peut conduire à des phénomènes de polymérisation radicalaire desdits composés monovinylaromatiques, notamment à température élevée. L'emploi d'inhibiteurs de polymérisation radicalaire, tels que phénols ou quinones, est souvent nécessaire ; toutefois la plupart de ces inhibiteurs ne sont pas suffisamment actifs à chaud et nécessitent la présence d'oxygène pour améliorer leur activité.

La Demanderesse a trouvé un procédé performant d'hydrosilylation des composés monovinylaromatiques, ne nécessitant pas la présence d'inhibiteur de réaction de polymérisation radicalaire, tout en écartant le risque d'une telle réaction de polymérisation.

Selon l'invention, il s'agit d'un procédé de préparation de polyorganosiloxanes à fonction(s) arylalkyle(s), de haute pureté, par réaction d'hydrosilylation d'un hydrogénopolyorganosiloxane, réactif (SiH), avec un composé monovinyl aromatique, réactif (Vi), en présence d'un catalyseur d'hydrosilylation, ledit procédé étant caractérisé en ce que
- l'opération d'hydrosilylation est réalisée
   * à une température de 50 à 150 °C, de préférence de 50 à 100 °C, tout particulièrement de 60 à 90°C,
   * en présence d'un solvant (S), inerte vis-à-vis des réactifs, présentant à pression atmosphérique un point d'ébullition inférieur à 200 °C, de préférence inférieur à 150°C,
   * par introduction simultanée des deux réactifs (Vi) et (SiH) dans le milieu réactionnel comprenant le solvant (S) et le catalyseur d'hydrosilylation, cette introduction étant réalisée de manière telle que les quantités respectives des deux réactifs (Vi) et (SiH) correspondent à un rapport molaire réactif (Vi) / réactif (SiH) de plus de 0,5 à 1,5 , de préférence de plus de 1 à 1,2 , et qu'à tout moment de la réaction d'hydrosilylation, la quantité de réactif (SiH) présente, exprimée en masse de fonctions SiH (29g pour 1 fonction), corresponde à moins de 2%, de préférence moins de 1% de la masse réactionnelle, en excluant la masse de solvant,
- et en ce que l'opération d'hydrosilylation est suivie d'une opération d'élimination du solvant (S), des réactifs résiduels (SiH) et (Vi) éventuels, et/ou d'une opération d'hydrogénation, ladite opération d'hydrogénation étant éventuellement suivie d'une opération d'élimination (ou d'une nouvelle opération d'élimination) des produits autres que les polyorganosiloxanes à fonction(s) arylalkyle(s).
   Dans la définition de la mole d'hydrogénopolyorganosiloxane, réactif (SiH), on considérera comme entité élémentaire la fonction -SiH.
   Dans la définition de la mole de composé monovinylaromatique, réactif (Vi), on considérera comme entité élémentaire la molécule-gramme de composé monovinylaromatique.
   Parmi les hydrogénopolyorganosiloxanes, réactifs (SiH) pouvant être mis en oeuvre pour réaliser le procédé de l'invention, on peut mentionner ceux répondant à la formule (1)

   (R¹) (R²) (R³) Si - O - [Si (R⁴) (H) - O]ₙ - [Si (R⁵) (R⁶) - O]ₘ - Si (R⁷) (R⁸) (R⁹) (I)

   ou leurs homologues cycliques, formule (I) dans laquelle
- les symboles R¹ et R⁹ sont semblables ou différents et représentent un atome d'hydrogène ou un radical alkyle contenant de 1 à 8 atomes de carbone, de préférence 1 ou 2 atomes de carbone, tout particulièrement 1 atome de carbone
- les symboles R², R³, R⁴, R⁵, R⁶, R⁷ et R⁸ sont semblables ou différents et représentent un radical alkyle contenant de 1 à 8 atomes de carbone, de préférence 1
ou 2 atomes de carbone, tout particulièrement 1 atome de carbone
. n est un nombre entier ou décimal pouvant aller de 0 à 10, de préférence de 0 à 5, au moins un des radicaux R¹ et R⁹ représentant un atome d'hydrogène lorsque n=0
. m est un nombre entier ou décimal pouvant aller de 0 à 50, de préférence de 0 à 10.

D'une manière préférentielle, n est égal à 1 et m est égal à 0 ; ledit réactif (SiH) est alors un hydrogénoheptaorganotrisiloxane (MD'M en abréviation), tout particulièrement l'hydrogénoheptaméthyltrisiloxane.

Parmi les composés monovinylaromatiques, réactifs (Vi), pouvant être mis en oeuvre pour réaliser le procédé de l'invention, on peut mentionner ceux de point d'ébullition inférieur à 200 °C, de préférence inférieur à 150°C, à pression atmosphérique et répondant à la formule (2)

CH₂=C(R) - Φ (R'¹) (R'²) (R'³) (R'⁴) (R'⁵) (2)

formule dans laquelle
. le symbole Φ représente un noyau aromatique contenant 6 atomes de carbone
. le symbole R représente H ou CH₃
. les symboles R'¹, R'², R'³, R'⁴ et R'⁵ sont semblables ou différents et représentent H, CH₃, F, Cl ou Br.

On peut citer d'une manière préférentielle, le styrène, l'α-méthylstyrène, le dimère de l'α-méthylstyrène, le pentafluorobenzène.

Parmi les solvants (S), inertes vis-à-vis des réactifs, pouvant être mis en oeuvre pour réaliser le procédé de l'invention, on peut citer des solvants organiques comme le toluène, le xylène, le méthylcyclohexane, l'heptane, l'octane ... et tout particulièrement les solvants polyorganosiloxanes, comme l'hexaméthyldisiloxane.

L'opération d'hydrosilylation est réalisée en présence d'un catalyseur d'hydrosilylation ; celui-ci est notamment choisi parmi ceux à base de platine (0) ou d'un dérivé du platine (0), tels que les complexes de platine décrits dans US-A-3,159,601, US-A-3,159,662, US-A-3,715,334, US-A-3,814,730 ... Un catalyseur préférentiel est le catalyseur de KARSTEDT, mis en oeuvre par exemple à raison de 1 à 300 parties, de préférence de 5 à 100 parties en masse de platine par million de parties en masse de réactifs (SiH) et (Vi) mis en oeuvre.

L'opération d'hydrosilylation est de préférence réalisée sous pression atmosphérique.

L'introduction des réactifs (SiH) et (Vi) est de préférence réalisée par coulée simultanée des deux réactifs en continu sur la masse réactionnelle comprenant le solvant et le catalyseur.
La durée des coulées est ajustée de façon à consommer le réactif (Vi) par hydrosilylation au fur et à mesure de son introduction. La quantité de réactif (Vi) dans le milieu est ainsi toujours faible, ce qui limite les risques de polymérisation radicalaire.
De même l'introduction simultanée des deux réactifs et leur consommation simultanée, permet d'éviter l'accumulation de fonctions -SiH libres dans le milieu ; l'exothermie potentielle de la réaction est ainsi limitée.
La mise en oeuvre d'un inhibiteur de polymérisation radicalaire n'est donc pas nécessaire.

Le solvant (S) et les réactifs non réagis peuvent ensuite éliminés. Leur élimination peut être réalisée par distillation sous vide ou pression réduite, (par exemple de l'ordre de 1,013 Pa à 101 300 Pa).

Cette opération de distillation peut éventuellement être suivie d'une opération d'hydrogénation, ce afin d'éliminer les insaturations restantes dues à une réaction de déshydrogénocondensation. La proportion de composés dont la présence est due à cette réaction de déshydrogénocondensation augmente avec la température. De tels composés sont indésirables dans des applications comme la cosmétologie ou la parfumerie notamment.

L'opération d'hydrogénation peut être réalisée à une température de l'ordre de 25 à 200°C, de préférence de l'ordre de 50 à 150°C, à une pression d'hydrogène de l'ordre de 0 à 50 bar, de préférence de l'ordre de 5 à 25 bar, en présence d'une quantité efficace de catalyseur d'hydrogénation.
Parmi les catalyseurs d'hydrogénation, on peut citer tout particulièrement le platine et le palladium ; ils peuvent être mis en oeuvre à raison de 0,01 à 5%, de préférence de 0,01 à 1% en poids de métal par rapport à la masse à hydrogéner. Les catalyseurs sont généralement déposés sur support de noir de carbone.

Selon une variante de réalisation (intéressante notamment pour la préparation de silicones à fonctions alkylaromatiques utilisés en cosmétique ou en parfumerie), le milieu issu de l'opération d'hydrosilylation est soumis à une opération d'hydrogénation selon les conditions décrites ci-dessus (afin de réduire la quantité ou d'éliminer la présence de composés insaturés résultant de la réaction d'hydrosilylation), puis est éventuellement soumis à une opération d'élimination des produits autres que les polyorganosiloxanes à fonction(s) arylalkyle(s). Cette opération d'élimination peut être réalisée par distillation sous vide ou pression réduite, par exemple de l'ordre de 1,013 Pa à 101 300 Pa.

Un mode particulier de réalisation du procédé faisant l'objet de l'invention, consiste à réaliser l'opération d'hydrosilylation, comme décrit ci-dessus, en mettant en oeuvre l'hydrogénoheptaméthyltrisiloxane comme réactif (SiH), du styrène comme réactif (Vi), et de l'hexaméthyldisiloxane comme solvant (S), à une température de 50 à 150°C, de préférence de 50 à 100°C, tout particulièrement de 60 à 90°C.
Cette opération est ensuite suivie d'une opération d'élimination sous vide du solvant (S) et des réactifs (SiH) et (Vi) restants, puis d'une opération d'hydrogénation et éventuellement d'une autre opération de distillation. L'ordre de réalisation des opérations de distillation et d'hydrogénation peut également être inversé.
L'opération d'hydrogénation peut ne pas être réalisée lorsque l'opération d'hydrosilylation a été effectuée à une température relativement basse, de l'ordre de 60°C par exemple, ou lorsque le réactif (Vi) mis en oeuvre ne conduit pas à la présence de composés insaturés (ceci est le cas de l'αméthylstyrène, notamment).

Le produit obtenu selon le procédé de l'invention, à partir d'hydrogénoheptaméthyltrisiloxane et de styrène, à une température d'hydrosilylation de 50 à 100°C, est formé d'un mélange constitué :
* pour plus de 70% massique, généralement pour au moins 75% massique de trisiloxane de formule

   Me₃ Si -O- Si (Me) (X) -O- Si Me₃,

   où X représente la fonction -CH₂-CH₂-Ph
* pour moins de 25% massique, généralement de 10 à 20 % massique, de trisiloxane de formule

   Me₃ Si -O- Si (Me) (Z) -O- Si Me₃,

   où Z représente la fonction -CH (CH₃) -Ph
* et moins de 5% massique, généralement de 0 à 2% massique de trisiloxane de formule

   Me₃ Si -O- Si (Me) (Y) -O- Si Me₃,

   où Y représente la fonction -CH=CH-Ph formules où Me représente un radical méthyle et Ph un radical phényle.

Les silicones à fonction(s) arylalkyle(s) de haute pureté obtenus selon le procédé de l'invention, notamment ceux à fonction(s) phénylalkyle(s), tout particulièrement ceux obtenus à partir d'hydrogénoheptaméthyltrisiloxane et de styrène, formés d'un mélange constitué :
* pour plus de 70% massique, généralement pour au moins 75% massique de trisiloxane de formule

   Me₃ Si -O- Si (Me) (X) -O- Si Me₃,

   où X représente la fonction -CH₂-CH₂-Ph
* pour moins de 25% massique, généralement de 10 à 20 % massique, de trisiloxane de formule

   Me₃ Si -O- Si (Me) (Z) -O- Si Me₃,

   où Z représente la fonction -CH (CH₃) -Ph
* et moins de 5% massique, généralement de 0 à 2% massique de trisiloxane de formule

   Me₃ Si -O- Si (Me) (Y) -O- Si Me₃,

   où Y représente la fonction -CH=CH-Ph formules où Me représente un radical méthyle et Ph représente un radical phényle, peuvent être notamment utilisés dans les compositions cosmétiques et les compositions parfumantes.

Ils peuvent être utilisés comme agents émollients et/ou véhicules et/ou agents anti-transfert dans les compositions cosmétiques. Ils peuvent constituer au moins partiellement le véhicule des substances actives naturelles ou synthétiques présentes dans les compositions cosmétiques ; ils peuvent ainsi remplacer au moins partiellement les véhicules lipophiles usuels. D'autre part, ils confèrent auxdites compositions des propriétés émollientes et peuvent ainsi remplacer au moins partiellement les agents émollients ou conditionneurs habituels, notamment les huiles silicones usuelles, comme notamment les organopolysiloxanes du type phényltriméthicone, octaméthyltetraméthicone.
Les quantités d'organotrisiloxane à fonction phénylalkyle ou phénylalkényle à utiliser, dépendent de la fonction recherchée et du type de formulation cosmétique considéré. L'homme de l'art est à même de déterminer ces quantités.
On peut mentionner comme suit, à titre indicatif les quantités en masse d'organotrisiloxane à fonction phénylalkyle favorablement présentes dans les compositions cosmétiques en fonction de l'application recherchée.

| | |
|---|---|
| produits de soin (crèmes, laits ...) | 0,5-5% |
| produits démaquillants | 1-2% |
| produits pour le corps | 0,5-1,5% |
| produits de maquillage (fonds de teint, poudres | jusqu'à 40% |
| compactes, mascaras, fards à paupières, rouges à lèvres) produits d'hygiène et de toilette (déodorants, | 1-10% |
| antiperspirants, produits de rasage et produits hydroalcooliques) produits solaires(crème, lait, huile) | jusqu'à 99%, voire plus, de préférence 10-40% |

| produits capillaires | |
|---|---|
| (shampoing, après shampoing) | jusqu'à 5% |
| (soin du cheveu, embellisseur du cheveu) | jusqu'à 10% |

Lesdits silicones à fonction(s) phénylalkyle(s), notamment ceux préparés à partir d'hydrogénoheptaméthyltrisiloxane et de styrène, peuvent être utilisés comme agents solubilisants et émollients dans les compositions parfumantes.
Lesdites compositions parfumantes peuvent comprendre de l'ordre de
- 3 à 20% de leur poids d'une base parfumante et
- 75 à 97% de leur poids d'un agent solubilisant lipophile à base desdits silicones à fonctions phénylalkyles.

La base parfumante présente peut être tout composé utilisé dans l'industrie de la parfumerie et responsable des diverses notes parfumées.
La composition parfumante est une solution, de préférence sans alcool (éthanol).
Selon une variante de réalisation, ledit agent solubilisant est constitué desdits silicones à fonctions phénylalkyles associés à au moins un autre solvant volatil ou non volatil des bases parfumantes, comme les silicones volatils (hexaméthyldisiloxane par exemple), le propylène glycol, les esters (dioctanoate diisononanoate de diéthylène glycol par exemple).
Ledit ou lesdits solvants autres peuvent être présents selon un rapport pondéral silicones à fonctions phénylalkyles / solvant(s) autre(s) de l'ordre de 5/95 à 95/5, de préférence de l'ordre de 10/90 à 90/10, tout particulièrement de l'ordre de 25/75 à 90/10.

Les exemples suivants sont donnés à titre illustratif.

### Exemple 1

### Hydrosilylation du styrène par l'heptaméthyltrisiloxane

Dans un réacteur de 10 litres, on introduit à l'aide d'une pompe 1803 g (11,12 moles) d'hexaméthyldisiloxane (HMDS) et 4,15 g d'une solution de platine de Karstedt titrant 11,5 % de platine (0). La masse réactionnelle est portée à 90°C et on coule en 5 heures de façon simultanée 4150 g (19,3 moles) d'heptaméthyltrisiloxane (MD'M) et 2207 g (21,22 moles) de styrène.
Le suivi des espèces majoritaires par chromatographie en phase gazeuse montre que la réaction est pratiquement quantitative (en % poids).

| **durée** | **HMDS** | **styrène** | **MD'M** | **X-HMTS** | **Y-HMTS** | **Z-HMTS** |
|---|---|---|---|---|---|---|
| 1 h | 54,9 | 0,6 | 1,5 | 30,4 | 0,8 | 5,6 |
| 2h | 36,9 | 2,0 | 1,6 | 42,9 | 1,7 | 7,8 |
| 3h | 30,3 | 2,3 | 2,1 | 47,7 | 2,5 | 8,3 |
| 4h | 24,2 | 2,1 | 2,8 | 53,2 | 3,2 | 9,3 |
| 5h | 21,7 | 3,2 | 2,5 | 53,2 | 3,3 | 9,3 |

La teneur en styrène libre dans la masse réactionnelle, en fin de réaction, représente 88% de l'excès de styrène engagé, ce qui est la preuve d'une polymérisation très faible.
Le complément à 100% est constitué par le produit de réaction des sous-produits du MD'M (MD'DM et MM' notamment) et du styrène.
Dans ce tableau,
* X-HMTS a la signification suivante

   Me₃ Si -O- Si (Me) (X) -O- Si Me₃,

   où X représente la fonction -CH₂-CH₂-Ph
* Y-HMTS a la signification suivante

   Me₃ Si -O- Si (Me) (Y) -O- Si Me₃,

   où Y représente la fonction -CH=CH-Ph
* Z-HMTS a la signification suivante

   Me₃ Si -O- Si (Me) (Z) -O- Si Me₃,

   où Z représente la fonction -CH (CH₃) -Ph avec Me représentant méthyle et Ph phényle.

### Distillation

La masse réactionnelle est alors concentrée (évaporation des volatils à 110°C sous 20 mbars pendant 7 heures). On recueille 5827 g d'un produit coloré de composition suivante (valeurs en % poids) :

| **HMDS** | **styrène** | **MD'M** | **X-HMTS** | **Y-HMTS** | **Z-HMTS** |
|---|---|---|---|---|---|
| 0,13 | 0,13 | 0,12 | 77,8 | 4,9 | 14,6 |

### Hydrogénation

Dans un réacteur autoclave de 1 litre , on charge 700g de ce produit coloré.
On introduit 14g (soit 2 % poids) d'un catalyseur platine sur noir titrant 2 % de Pt. La masse réactionnelle est portée à 100°C sous une pression de 20 bar d'hydrogène. Au bout de trois heures de réaction sous agitation , la masse réactionnelle est refroidie et ramenée sous pression atmosphérique. On obtient après filtration 692 g d'un produit incolore de composition suivante :

| **HMDS** | **styrène** | **MD'M** | **X-HMTS** | **Y-HMTS** | **Z-HMTS** |
|---|---|---|---|---|---|
| 0,13 | 0 | 0,1 | 80,75 | 1,10 | 14,3 |

Après distillation sur une colonne garnie d'anneaux Rashig, (hauteur = 40 cm), on recueille 563,3 g (rendement = 81,4 %) d'un mélange de composition suivante :

| **HMDS** | **styrène** | **MD'M** | **X-HMTS** | **Y-HMTS** | **Z-HMTS** |
|---|---|---|---|---|---|
| 0 | 0 | 0 | 81,5 | 1,04 | 16,6 |

### Exemple 2

### Hydrosilylation du styrène par l'heptaméthyltrisiloxane

Dans un réacteur de 1 I, on introduit à l'aide d'une pompe 178,9 g (1 mole) d'hexaméthyldisiloxane et 0,2708 g d'une solution de platine de Karstedt titrant 11,5 % de platine. La masse réactionnelle est portée à 60°C et on coule en 5 heures de façon simultanée 250,7 g (1,127 moles) d'heptaméthyltrisiloxane (OSI) et 128.9 g (1,24 moles) de styrène.
En fin de coulée la composition de la masse réactionnelle analysée par chromatographie en phase gazeuse est la suivante (en % poids) :

| **HMDS** | **styrène** | **MD'M** | **X-HMTS** | **Y-HMTS** | **Z-HMTS** |
|---|---|---|---|---|---|
| 32,9 | 2,69 | 0 | 49,6 | 0,65 | 13,15 |

La teneur en styrène libre correspond à 96% de l'excès de styrène engagé.
On recueille 525,3 g d'un produit coloré noir soit un bilan matière de 97 %.

### Distillation

La distillation de ce brut réactionnel sur une colonne garnie d'anneaux Rashig, (de 40 cm de hauteur) conduit à 305,5 g (soit un rendement de 76,4%) d'un mélange de composition suivante :

| **HMDS** | **styrène** | **MD'M** | **X-HMTS** | **Y-HMTS** | **Z-HMTS** |
|---|---|---|---|---|---|
| 0 | 0 | 0 | 77,48 | 0,96 | 21,25 |

On constate que la teneur en dérivé insaturé Y-HMTS est voisine de celle obtenue à l'exemple précédent réalisé à 90°C (hydrosilylation) et incluant une opération d'hydrogénation.
Une opération d'hydrosilylation réalisée à plus basse température permet donc de diminuer sensiblement la teneur en composé insaturé Y-HMTS.

### Exemple 3

### Hydrosilylation de l'heptaméthyltisiloxane et de l'α-méthylstyrène

Dans un réacteur de 2 I, on introduit à l'aide d'une pompe 337 g (2,08 moles) d'hexaméthyldisiloxane (HMDS) et 0,52 g d'une solution de platine de Karstedt titrant 11,5 % de platine. La masse réactionnelle est portée à 90°C et on coule en 5 heures de façon simultanée 444 g (2 moles) d'heptaméthyltrisiloxane (MD'M) et 259,6 g (2,2 moles) d'α-méthylstyrène.
Au bout de 24 h, la composition de la masse réactionnelle analysée par chromatographie en phase gazeuse est la suivante (en % poids) :

| **HMDS** | **α-méthylstyrène** | **MD'M** | **M-HMTS** |
|---|---|---|---|
| 30,1 | 5,17 | 4,36 | 60,8 |

Le complément à 100% est constitué par le produit de réaction des sous-produits du MD'M (MD'DM et MM' notamment) et d'α-méthylstyrène.
Dans ce tableau,
* M-HMTS a la signification suivante
   Me₃ Si -O- Si (Me) (X) -O- Si Me₃, où M représente la fonction -CH (CH₃) -CH₂-Ph
   Après distillation, on obtient 569,9 g d'un produit titrant 96 % de pureté du composé principal (rendement = 81 %)

### Exemple 4

### Composition parfumante

. Lavandin 7% en poids
. silicone à fonction phénylalkyle des exemples 1, 2 ou 3 93% en poids
Cette composition est obtenue par simple mélange de ses deux constituants

### Exemple 5

### Composition parfumante

. Lavandin 15% en poids
. silicone à fonction phénylalkyle des exemples 1, 2 ou 3 50% en poids
. hexaméthyldisiloxane 35% en poids
Cette composition est obtenue par simple mélange de ses trois constituants.

### Exemple 6

### Déodorant sous forme de stick transparent

| | |
|---|---|
| silicone à fonction phénylalkyle des exemples 1, 2 ou 3 | 9% |
| myristyl ester polypropoxylé de l'acide propionique | 70% |
| propylène glycol | 14% |
| triclosan | 0,3% |
| stéarate de sodium | 6% |
| parfum | qsp |

### Exemple 7

### Huile solaire sèche

| | |
|---|---|
| silicone à fonction phénylalkyle des exemples 1, 2 ou 3 | 27% |
| isopropylpalmitate | 25% |
| diisopropyladipate | 25% |
| Mirasil C-DPDM | 20% |
| octylméthoxycinnamate | 3% |

Cette composition est obtenue par simple mélange de ses constituants.

### Exemple 8

### Lotion capillaire pour le traitement des pointes de cheveux

| | |
|---|---|
| silicone à fonction phénylalkyle des exemples 1, 2 ou 3 | 5% |
| Mirasil C-DPDM | 90% |
| éthanol | 5% |

Cette composition est obtenue par simple mélange de ses constituants.

## Revendications

1. Procédé de préparation de polyorganosiloxanes à fonction(s) arylalkyle(s), de haute pureté, par réaction d'hydrosilylation d'un hydrogénopolyorganosiloxane, réactif (SiH), avec un composé monovinyl aromatique, réactif (Vi), en présence d'un catalyseur d'hydrosilylation, ledit procédé étant **caractérisé en ce que**
- l'opération d'hydrosilylation est réalisée
* à une température de 50 à 150 °C, de préférence de 50 à 100 °C, tout particulièrement de 60 à 90°C,
* en présence d'un solvant (S), inerte vis-à-vis des réactifs, présentant à pression atmosphérique un point d'ébullition inférieur à 200 °C, de préférence inférieur à 150°C,
* par introduction simultanée des deux réactifs (Vi) et (SiH) dans le milieu réactionnel comprenant le solvant (S) et le catalyseur d'hydrosilylation, cette introduction étant réalisée de manière telle que les quantités respectives des deux réactifs (Vi) et (SiH) correspondent à un rapport molaire réactif (Vi) / réactif (SiH) de plus de 0,5 à 1,5, de préférence de plus de 1 à 1,2 , et qu'à tout moment de la réaction d'hydrosilylation, la quantité de réactif (SiH) présente, exprimée en masse de fonctions SiH (29g pour 1 fonction), corresponde à moins de 2%, de préférence moins de 1% de la masse réactionnelle, en excluant la masse de solvant,
- et **en ce que** l'opération d'hydrosilylation est suivie d'une opération d'élimination du solvant (S), des réactifs résiduels (SiH) et (Vi) éventuels, et/ou d'une opération d'hydrogénation, ladite opération d'hydrogénation étant éventuellement suivie d'une opération d'élimination (ou d'une nouvelle opération d'élimination) des produits autres que les polyorganosiloxanes à fonction(s) arylalkyle(s).

2. Procédé selon la revendication 1), **caractérisé en ce que** le réactif (SiH) est choisi parmi les réactifs de formule (1)
(R¹) (R²) (R³) Si - O - [Si (R⁴) (H) - O]ₙ - [Si (R⁵) (R⁶)- O]ₘ - Si (R⁷) (R⁸) (R⁹) (I)
ou leurs homologues cycliques, formule (I) dans laquelle
. les symboles R¹ et R⁹ sont semblables ou différents et représentent un atome d'hydrogène ou un radical alkyle contenant de 1 à 8 atomes de carbone, de préférence 1 ou 2 atomes de carbone, tout particulièrement 1 atome de carbone
. les symboles R², R³, R⁴, R⁵, R⁶, R⁷ et R⁸ sont semblables ou différents et représentent un radical alkyle contenant de 1 à 8 atomes de carbone, de préférence 1 ou 2 atomes de carbone, tout particulièrement 1 atome de carbone
. n est un nombre entier ou décimal pouvant aller de 0 à 10, de préférence de 0 à 5, au moins un des radicaux R¹ et R⁹ représentant un atome d'hydrogène lorsque n=0
. m est un nombre entier ou décimal pouvant aller de 0 à 50, de préférence de 0 à 10.

3. Procédé selon la revendication 2), **caractérisé en ce que** n est égal à 1 et m est égal à 0.

4. Procédé selon la revendication 3), **caractérisé en ce que** ledit réactif (SiH) est l'hydrogénoheptaméthyltrisiloxane.

5. Procédé selon l'une quelconque des revendications 1) à 4), **caractérisé en ce que** le réactif (Vi) est choisi parmi ceux de point d'ébullition inférieur à 200 °C, de préférence inférieur à 150°C, à pression atmosphérique et répondant à la formule (2).
CH₂=C(R) - Φ (R'¹) (R'²) (R'³) (R'⁴) (R'⁵) (2)
formule dans laquelle
. le symbole Φ représente un noyau aromatique contenant 6 atomes de carbone
. le symbole R représente H ou CH₃
. les symboles R'¹, R'², R'³, R'⁴ et R'⁵ sont semblables ou différents et représentent H, CH₃, F, Cl ou Br.

6. Procédé selon la revendication 5) **caractérisé en ce que** le réactif (Vi) est choisi parmi le styrène, l'α-méthylstyrène, le dimère de l'α-méthylstyrène, le pentafluorobenzène.

7. Procédé selon l'une quelconque des revendications 1) à 6), **caractérisé en ce que** le solvant (S) est choisi parmi les solvants organiques comme le toluène, le xylène, le méthylcyclohexane, l'heptane, l'octane, et tout particulièrement les solvants polyorganosiloxanes, comme l'hexaméthyldisiloxane.

8. Procédé selon l'une quelconque des revendications 1) à 7), **caractérisé en ce que** l'introduction des réactifs (SiH) et (Vi) est réalisée par coulée simultanée des deux réactifs en continu sur la masse réactionnelle comprenant le solvant et le catalyseur.

9. Procédé selon l'une quelconque des revendications 1) à 8), **caractérisé en ce que** le solvant (S) et les réactifs non réagis lors de l'opération d'hydrosilylation sont éliminés par distillation sous vide ou pression réduite, et **en ce que** l'opération de distillation est suivie d'une opération d'hydrogénation.

10. Procédé selon l'une quelconque des revendications 1) à 8), **caractérisé en ce que** le milieu issu de l'opération d'hydrosilylation est soumis à une opération d'hydrogénation, puis éventuellement à une opération d'élimination des produits autres que les polyorganosiloxanes à fonction(s) arylalkyle(s) par distillation sous vide ou pression réduite.

11. Procédé selon la revendication 1), **caractérisé en ce que** l'opération d'hydrosilylation est réalisée en mettant en oeuvre l'hydrogénoheptaméthyltrisiloxane comme réactif (SiH), du styrène comme réactif (Vi), et de l'hexaméthyldisiloxane comme solvant (S), à une température de 50 à 150°C, et **en ce qu'**elle est suivie d'une opération d'élimination sous vide du solvant (S) et des réactifs (SiH) et (Vi) restants, puis d'une opération d'hydrogénation et éventuellement d'une autre opération de distillation, l'ordre de réalisation des opérations de distillation et d'hydrogénation pouvant être inversé.

12. Procédé selon la revendication 11), **caractérisé en ce que** l'opération d'hydrosilylation est réalisée à une température de 50 à 100°C, tout particulièrement de 60 à 90°C.

13. Produit obtenu selon le procédé de la revendication 12), **caractérisé en ce qu'**il est formé d'un mélange constitué :
* pour plus de 70% massique, généralement pour au moins 75% massique de trisiloxane de formule
Me₃ Si -O- Si (Me) (X) -O- Si Me₃,
où X représente la fonction -CH₂-CH₂-Ph
* pour moins de 25% massique, généralement de 10 à 20 % massique, de trisiloxane de formule
Me₃ Si -O- Si (Me) (Z) -O- Si Me₃,
où Z représente la fonction -CH (CH₃) -Ph
* et moins de 5% massique, généralement de 0 à 2% massique de trisiloxane de formule
Me₃ Si -O- Si (Me) (Y) -O- Si Me₃,
où Y représente la fonction -CH=CH-Ph formules où Me représente un radical méthyle et Ph un radical phényle.

## Claims

1. Process for preparing polyorganosiloxanes containing (an) arylalkyl function(s), of high purity, by hydrosilylation reaction of a hydrogenopolyorganosiloxane, reagent (SiH), with a monovinylaromatic compound, reagent (Vi), in the presence of a hydrosilylation catalyst, the said process being **characterized in that**
- the hydrosilylation operation is carried out
* at a temperature from 50°C to 150°C, preferably from 50°C to 100°C and most particularly from 60°C to 90°C,
* in the presence of a solvent (S), which is inert with respect to the reagents, having a boiling point at atmospheric pressure of less than 200°C, preferably less than 150°C,
* by simultaneous introduction of the two reagents (Vi) and (SiH) into the reaction medium comprising the solvent (S) and the hydrosilylation catalyst, this introduction being carried out such that the respective amounts of the two reagents (Vi) and (SiH) corresponding to a reagent (Vi)/reagent (SiH) molar ratio of more than 0.5 to 1.5, preferably of more than 1 to 1.2, and **in that** at any moment in the hydrosilylation reaction, the amount of reagent (SiH) present, expressed as the mass of SiH functions (29 g per 1 function), corresponds to less than 2% and preferably less than 1% of the reaction mass, excluding the mass of solvent,
- and **in that** the hydrosilylation operation is followed by an operation for removal of the solvent (S) and any possible residual reagents (SiH) and (Vi), and/or by a hydrogenation operation, the said hydrogenation operation optionally being followed by an operation for removal (or a new operation for removal) of the products other than the polyorganosiloxanes containing (an) arylalkyl function(s).

2. Process according to Claim 1, **characterized in that** the reagent (SiH) is chosen from the reagents of formula (1)
(R¹) (R²) (R³)Si-O-[Si(R⁴)(H)-O]ₙ-[Si(R⁵) (R⁶)-O]ₘ-Si(R⁷) (R⁸) (R⁹) (1)
or cyclic homologues thereof, of formula (1) in which
· the symbols R¹ and R⁹ are identical or different and represent a hydrogen atom or an alkyl radical containing from 1 to 8 carbon atoms, preferably 1 or 2 carbon atoms and most particularly 1 carbon atom,
· the symbols R², R³, R⁴, R⁵, R⁶, R⁷ and R⁸ are identical or different and represent an alkyl radical containing from 1 to 8 carbon atoms, preferably 1 or 2 carbon atoms and most particularly 1 carbon atom,
· n is an integer or decimal number which may be from 0 to 10, preferably from 0 to 5, at least one of the radicals R¹ and R⁹ representing a hydrogen atom when n=0,
· m is an integer or decimal number which may be from 0 to 50, preferably from 0 to 10.

3. Process according to Claim 2, **characterized in that** n is equal to 1 and m is equal to 0.

4. Process according to Claim 3, **characterized in that** the said reagent (SiH) is hydrogenoheptamethyltrisiloxane.

5. Process according to any one of Claims 1 to 4, **characterized in that** the reagent (Vi) is chosen from those with a boiling point of less than 200°C, preferably less than 150°C, at atmospheric pressure and corresponding to formula (2)
CH₂=C(R)-Φ(R'¹) (R'²) (R'³) (R'⁴) (R'⁵) (2)
in which formula
· the symbol Φ represents an aromatic nucleus containing 6 carbon atoms,
· the symbol R represents H or CH₃,
· the symbols R'¹, R'², R'³, R'⁴ and R'⁵ are identical or different and represent H, CH₃, F, Cl or Br.

6. Process according to Claim 5, **characterized in that** the reagent (Vi) is chosen from styrene, α-methylstyrene, α-methylstyrene dimer and pentafluorobenzene.

7. Process according to any one of Claims 1 to 6, **characterized in that** the solvent (S) is chosen from organic solvents such as toluene, xylene, methylcyclohexane, heptane, octane and, most particularly, polyorganosiloxane solvents such as hexamethyldisiloxane.

8. Process according to any one of Claims 1 to 7, **characterized in that** the introduction of the reagents (SiH) and (Vi) is carried out by simultaneously and continuously adding the two reagents to the reaction mass comprising the solvent and the catalyst.

9. Process according to any one of Claims 1 to 8, **characterized in that** the solvent (S) and the unreacted reagents from the hydrosilylation operation are removed by distillation under vacuum or reduced pressure, and **in that** the distillation operation is followed by a hydrogenation operation.

10. Process according to any one of Claims 1 to 8, **characterized in that** the medium obtained from the hydrosilylation operation is subjected to a hydrogenation operation, and then optionally to an operation to remove the products other than the polyorganosiloxanes containing (an) arylalkyl function(s) by distillation under vacuum or reduced pressure.

11. Process according to Claim 1, **characterized in that** the hydrosilylation operation is carried out using hydrogenoheptamethyltrisiloxane as reagent (SiH), styrene as reagent (Vi) and hexamethyldisiloxane as solvent (S), at a temperature from 50°C to 150°C, and **in that** it is followed by an operation for removal under vacuum of the solvent (S) and of the remaining reagents (SiH) and (Vi), followed by a hydrogenation operation and optionally by another distillation operation, the order in which the distillation and hydrogenation operations is carried out possibly being reversed.

12. Process according to Claim 11, **characterized in that** the hydrosilylation operation is carried out at a temperature from 50°C to 100°C, most particularly from 60°C to 90°C.

13. Product obtained according to the process of Claim 12, **characterized in that** it is formed from a mixture consisting:
* for more than 70% by mass, generally for at least 75% by mass, of trisiloxane of formula
Me₃Si-O-Si(Me) (X)-O-SiMe₃,
in which X represents a -CH₂-CH₂-Ph function,
* for less than 25% by mass, generally from 10% to 20% by mass, of trisiloxane of formula
Me₃Si-O-Si(Me) (Z)-O-SiMe₃,
in which Z represents a -CH(CH₃)-Ph function,
* and less than 5% by mass, generally from 0% to 2% by mass, of trisiloxane of formula
Me₃Si-O-Si(Me) (Y)-O-SiMe₃,
in which Y represents a -CH=CH-Ph function,
in which formulae Me represents a methyl radical and Ph represents a phenyl radical.

## Patentansprüche

1. Verfahren zur Herstellung von Polyorganosiloxanen mit Arylalkyl-Funktion(en) von hoher Reinheit durch Reaktion der Hydrosilylierung eines Hydrogenpolyorganosiloxans, Reaktand (SiH), mit einer aromatischen Monovinyl-Verbindung, Reaktand (Vi), in Anwesenheit eines Katalysators der Hydrosilylierung, **dadurch gekennzeichnet, daß**
- die Operation der Hydrosilylierung realisiert wird
* bei einer Temperatur von 50 °C bis 150 °C, vorzugsweise von 50 °C bis 100 °C und noch bevorzugter von 60 °C bis 90 °C,
* in Anwesenheit eines Lösungsmittels (S), das gegenüber den Reaktanden inert ist und bei atmosphärischem Druck einen Siedepunkt von unter 200 °C, vorzugsweise von unter 150 °C aufweist,
* durch gleichzeitiges Eintragen der beiden Reaktanden (Vi) und (SiH) in das Reaktionsmedium, das das Lösungsmittel (S) und den Katalysator der Hydrosilylierung umfaßt, wobei dieses Eintragen in der weise realisiert wird, daß die jeweiligen Mengen an den beiden Reaktanden (Vi) und (SiH) einem molaren Verhältnis Reaktand (Vi)/Reaktand (SiH) von mehr als 0,5 bis 1,5, vorzugsweise mehr als 1 bis 1,2 entsprechen, und daß in jedem Moment der Reaktion der Hydrosilylierung die Menge von anwesendem Reaktanden (SiH), ausgedrückt in Masse der Funktionen SiH (29 g pro 1 Funktion), weniger als 2 %, vorzugsweise weniger als 1 % der Reaktionsmasse entspricht, ausschließlich der Masse von Lösungsmittel,
- und dadurch, daß sich an die Reaktion der Hydrosilylierung eine Operation zur Entfernung des Lösungsmittels (S), der restlichen Reaktanden (SiH) und gegebenenfalls (Vi) und/oder eine Operation der Hydrierung anschließt, wobei die genannte Operation der Hydrierung gegebenenfalls von einer Operation zur Entfernung (oder von einer neuen Operation zur Entfernung) der Produkte gefolgt wird, die anders sind als die Polyorganosiloxane mit Arylalkyl-Funktion(en).

2. Verfahren nach Anspruch 1, **dadurch gekennzeichnet, daß** der Reaktand (SiH) ausgewählt wird unter den Reaktanden der Formel (1)
(R¹) (R²) (R³)Si-O-[Si(R⁴) (H)-O]ₙ-[Si(R⁵)(R⁶)-O]ₘ-Si(R⁷)(R⁸)(R⁹) (1)
oder ihren cyclischen Homologen, wobei in der Formel (1)
. die Symbole R¹ und R⁹ gleich oder verschieden sind und ein wasserstoffatom oder einen Rest Alkyl mit 1 bis 8 Kohlenstoffatomen, vorzugsweise 1 oder 2 Kohlenstoffatomen, ganz besonders 1 Kohlenstoffacom, darstellen,
. die Symbole R², R³, R⁴, R⁵, R⁶, R⁷ und R⁸ gleich oder verschieden sind und einen Rest Alkyl mit 1 bis 8 Kohlenstoffatomen, vorzugsweise 1 oder 2 Kohlenstoffatomen, ganz besonders 1 Kohlenstoffatom, darstellen,
. n eine ganze oder dezimale Zahl von 0 bis 10, vorzugsweise von 0 bis 5 ist und mindestens einer der Reste R¹ und R⁹ ein Wasserstoffatom bedeutet, wenn n = 0 ist,
. m eine ganze oder dezimale Zahl von 0 bis 50, vorzugsweise von 0 bis 10 ist.

3. Verfahren nach Anspruch 2, **dadurch gekennzeichnet, daß** n gleich 1 ist und m gleich 0 ist.

4. Verfahren nach Anspruch 3, **dadurch gekennzeichnet, daß** der Reaktand (SiH) Hydrogenheptamethyltrisiloxan ist.

5. Verfahren nach irgendeinem der Ansprüche 1 bis 4, **dadurch gekennzeichnet, daß** der Reaktand (Vi) unter denen ausgewählt wird, die einen Siedepunkt von unter 200 °C, vorzugsweise von unter 150 °C, bei atmosphärischem Druck aufweisen und der Formel (2)
CH₂=C(R)-Φ(R'¹)(R'²)(R'³)(R'⁴) (R'⁵) (2)
entsprechen, in der
. das Symbol Φ einen aromatischen Kern mit 6 Kohlenstoffatomen darstellt,
. das Symbol R H oder CH₃ bedeutet,
. die Symbole R'¹, R'², R'³, R'⁴ und R'⁵ gleich oder verschieden sind und H, CH₃, F, Cl oder Br darstellen.

6. Verfahren nach Anspruch 5, **dadurch gekennzeichnet, daß** der Reaktand (Vi) ausgewählt wird unter Styrol, α-Methylstyrol, dem Dimer von α-Methylstyrol und Pentafluorbenzol.

7. Verfahren nach irgendeinem der Ansprüche 1 bis 6, **dadurch gekennzeichnet, daß** das Lösungsmittel (S) ausgewählt wird unter den organischen Lösungsmitteln wie Toluol, Xylol, Methylcyclohexan, Heptan, Octan und ganz besonders den Polyorganosiloxan-Lösungsmitteln wie Hexamethyldisiloxan.

8. Verfahren nach irgendeinem der Ansprüche 1 bis 7, **dadurch gekennzeichnet, daß** das Eintragen der Reakcanden (SiH) und (Vi) durch gleichzeitiges kontinuierliches Zugießen der beiden Reaktanden zu der Reaktionsmasse, die das Lösungsmittel und den Katalysator umfaßt, realisiert wird.

9. Verfahren nach irgendeinem der Ansprüche 1 bis 8, **dadurch gekennzeichnet, daß** das Lösungsmittel (S) und die bei der Reaktion der Hydrosilylierung nicht umgesetzten Reaktanden durch Destillation unter Vakuum oder unter reduziertem Druck entfernt werden, und daß auf die Operation der Destillation eine Operation der Hydrierung folgt.

10. Verfahren nach irgendeinem der Ansprüche 1 bis 8, **dadurch gekennzeichnet, daß** das von der Operation der Hydrosilylierung stammende Medium einer Operation der Hydrierung unterzogen wird und anschließend gegebenenfalls einer Operation zur Entfernung der Produkte, die anders sind als die Polyorganosiloxane mit Arylalkyl-Funktion(en), durch Destillation unter Vakuum oder unter reduziertem Druck.

11. Verfahren nach Anspruch 1, **dadurch gekennzeichnet, daß** die Operation der Hydrosilylierung unter Einsatz von Hydrogenheptamethyltrisiloxan als Reaktand (SiH), Styrol als Reaktand (Vi) und Hexamethyldisiloxan als Lösungsmittel (S) bei einer Temperatur von 50 °C bis 150 °C realisiert wird, und daß sie von einer Operation zur Entfernung des Lösungsmittels (S) und der restlichen Reaktanden (SiH) und (Vi), sowie anschließend von einer Operation der Hydrierung und gegebenenfalls von einer anderen Operation der Destillation gefolgt wird, wobei die Reihenfolge der Operationen von Destillation und Hydrierung auch umgekehrt sein kann.

12. Verfahren nach Anspruch 11, **dadurch gekennzeichnet, daß** die Operation der Hydrosilylierung bei einer Temperatur von 50 °C bis 100 °C, ganz besonders von 60 °C bis 90 °C, realisiert wird.

13. Produkt, erhalten nach dem Verfahren von Anspruch 12, **dadurch gekennzeichnet, daß** es aus einer Mischung gebildet wird, bestehend aus:
* mehr als 70 Masse-%, im allgemeinen mehr als 75 Masse-% Trisiloxan der Formel Me₃Si-O-Si(Me) (X)-O-SiMe₃, worin X die Funktion -CH₂-CH₂-Ph darstellt,
* weniger als 25 Masse-%, im allgemeinen 10 bis 20 Masse-% Trisiloxan der Formel Me₃Si-O-Si(Me) (Z)-O-SiMe₃, worin Z die Funktion -CH(CH₃)-Ph darstellt, und
* weniger als 5 Masse-%, im allgemeinen 0 bis 2 Masse-% Trisiloxan der Formel Me₃Si-O-Si(Me)(Y)-O-SiMe₃, worin Y die Funktion -CH=CH-Ph darstellt,
und wobei in den Formeln Me einen Rest Methyl bedeutet und Ph einen Rest Phenyl darstellt.
